# EUROPEAN PATENT SPECIFICATION

(11) **EP 3 335 683 B1**
(45) Date of publication and mention of the grant of the patent: **20.01.2021**
(21) Application number: 16205124.7
(22) Date of filing: 19.12.2016
(51) Int. Cl.: A61F 13/474, A61F 13/476, A61F 13/15, A61F 13/56

(54) **DISPOSABLE ABSORBENT ARTICLE HAVING UNDERGARMENT FASTENING ELEMENTS**
SAUGFÄHIGER EINWEGARTIKEL MIT UNTERWÄSCHEBEFESTIGUNGSELEMENTEN
ARTICLE ABSORBANT JETABLE COMPORTANT DES ÉLÉMENTS DE FIXATION AU SOUS-VÊTEMENT

(43) Date of publication of application: 20.06.2018
(73) Proprietor: The Procter & Gamble Company, Cincinnati, OH 45202 (US)
(72) Inventor: LANGDON, Frederick Michael, Cincinnati, Ohio 45202 (US); BELLUCCI, Remo, Cincinnati, Ohio 45202 (US); LAVASH, Bruce William, Cincinnati, Ohio 45202 (US); WILLIAMS LUDHER, Callida Ann, Cincinnati, Ohio 45202 (US); STEVENS, Douglas Gregory, Cincinnati, Ohio 45202 (US)
(74) Representative: P&G Patent Germany

(56) References cited:
- WO-A2-94/14398
- US-A1- 2015 351 978

## Description

### FIELD OF THE INVENTION

This invention relates to absorbent articles such as catamenial devices and light incontinence products. In particular, this invention relates to catamenial devices such as sanitary napkins e.g. undergarment/panty liners having improved comfort and body fit.

### BACKGROUND OF THE INVENTION

Sanitary napkins and related disposable absorbent articles that provide for the collection of menses and other bodily discharges are well known in the art.

Such sanitary napkins fit within the undergarments of a user, and may have side flaps attached to their absorbent chassis which can adhere to the undergarments by an adhesive.

However, the sanitary napkin and in particular side flaps are often subjected to stresses during use. For example, the movement of the wearer, e.g. standing, sitting, running, etc., can cause the pressure sensitive adhesive to release from the undergarment and cause discomfort for the wearer. Unfortunately, often times the design of the sanitary napkin further contributes to the stresses applied to the side flaps during use further compromising the fit / comfort of the sanitary napkin.

Accordingly, there is a need for a sanitary napkin having side flaps configured to facilitate the adherence of the sanitary napkin to the undergarment of the user. Additionally, there is a need for a sanitary napkin having side flaps which improves the fit / comfort to the wearer while reducing stresses on the side flaps during use. Also there is a need for a sanitary napkin having side flaps which can be manufactured easily.

### SUMMARY OF THE INVENTION

Absorbent articles constructed in accordance with the present invention can facilitate the application of the absorbent article to an undergarment of a user as described herein.

In a first aspect, the invention provides an absorbent article comprising:
a chassis comprising a topsheet, a backsheet, and an absorbent core disposed between the topsheet and the backsheet, the chassis having a body-facing surface and an outer-facing surface, and
a pair of side flaps comprising a first surface, a second surface opposite the first surface, and a first adhesive,
each of the side flaps having a proximal end and a free distal end,
wherein each distal end is foldable around an edge of an undergarment when applied on the undergarment during use to attach the first adhesive to the underside of the undergarment thereby attaching the absorbent article to the undergarment,
wherein each of the side flaps is formed discretely to the chassis and is bonded at the proximal end by the first surface to the body facing surface of the chassis at a joint,
and wherein each side flap extends inwards from the joint in a lateral direction of the absorbent article with the first surface facing the top sheet of the absorbent article,
and wherein each side flap is extensible.

Each of the side flaps is formed discretely to the chassis. In other words, the side flaps are formed from a first piece of material and the chassis is formed from a second piece of material, and then the side flaps are bonded to the to the body facing surface of the chassis, so that they face inward i.e. towards the centre of the absorbent article.

In use, the side flaps are folded about the joint, such that the distal ends and the first surface pivot away from the body facing surface of the chassis and wrap around the undergarment, where they affix to the underside of the undergarment, thereby attaching the absorbent article to the undergarment.

In one embodiment the two side flaps comprise a single piece of material when presented to the user and can be separated in use to provide the two discrete flaps, for example by breaking a line of perforation.

The joint generated where the side flaps are bonded to the upper surface of the chassis allows easy folding of the side flaps and a good fit around the undergarment.

Because the side flaps are formed discretely to the chassis, the chassis and side flaps can comprise different materials. The side flaps are formed of a material which is extensible. In use, the side flaps usually overlay the undergarment near or on top of the leg elastic which is an area of the undergarment that is subjected to large strains since it wraps the leg and is in a "high motion zone" during wear.

The chassis has a lateral edge and the joint is spaced from the lateral edge over at least a part of a longitudinal extent of the joint. In other words, there is a gap between the lateral edge and the joint. Therefore, in use when the side flaps are folded around the undergarment, the extensible material is in contact with both faces of the undergarment

In one embodiment, the joint is spaced from the lateral edge over all the longitudinal extent of the joint.

In one embodiment, the joint is substantially parallel to the lateral edge i.e. the distance between the lateral edge and the joint is substantially uniform or substantially constant.

In one embodiment, the joint is spaced from the lateral edge by between 3 mm and 10 mm, for example between 3, 4, or 5 mm and 8, 9 or 10 mm.

The side flaps may be bonded to the body facing surface mechanically, ultrasonically, thermally, by pressure, cohesively or adhesively, and in a manner which is continuous or intermittent.

Because the side flaps are formed separately and then attached to the top of the absorbent article, there is no need for an additional process step of back folding the side flaps to the top of the absorbent article during manufacture. The side flaps may simply be placed on the body facing surface of the absorbent article.

In one embodiment, the side flaps are extensible in the longitudinal direction of the absorbent article. If stretch is allowed in this area, it will make the undergarment less binding and more comfortable to the wearer because the bonded area will not prevent longitudinal stretch of the undergarment.

In one embodiment, the side flaps are made extensible by mechanical activation.

In one embodiment, the mechanical activation is provided by a so-called SELF-pattern.

The terms "SELF" and "SELF'ing", refer to Procter & Gamble technology in which SELF stands for Structural Elastic Like Film. The process was originally developed for deforming a polymer film which has a low or no elasticity to provide it with elastic properties, and zones with differential extensibility. The deformation is typically mechanical and can be obtained e.g. ringrolling the material according to a predetermined SELFing pattern.. It has been found that the SELF'ing process can be also applicable to other materials such as film laminates nonwovens and film/nowoven laminates such as those constituting the wings of absorbent articles. Processes, apparatuses, and patterns produced via SELF are illustrated and described in U.S. Pat. Nos.: 5,518,801; 5,691,035; 5,723,087; 5,891,544; 5,916,663; 6,027,483; and 7,527,615 B2.

Absorbent articles having SELFed wings where the extensibility provided by SELFing is used to provide the wings with stress relief means are described in US patent application US2004068244A1. The distribution and configuration of SELFing patters described in this application are applicable, mutatis mutandis, also to the wings in the present invention.

A SELF-pattern is particularly suitable because it has low extensive forces and a low elastic recovery force.

In one embodiment, each of the side flaps comprises a laminate of a nonwoven and a polyethylene film and are made extensible by a SELF-pattern embossed onto the laminate. Such laminates are advantageous because they provide impermeability and softness.

The SELF-pattern may be curved or linear. In one embodiment, the SELF-pattern is curved to provide in use a visual cue that the absorbent article is aligned with the edge of the undergarment. The edge will typically be curved to fit the leg of a user.

In one embodiment, the joint is curved. Because the side flaps and topsheet are formed separately, the shape of the joint can be easily profiled to a curve by cutting appropriately shaped side flaps and top sheets. When the flaps fold under and affix to the undergarment, the fold forms substantially around the joint between the topsheet and side flap. Thereby, because the curve between the side flap and topsheet can be precisely controlled, the absorbent article of the invention fits the undergarment more closely. In addition, the curved joint provides the wearer with an alignment guide for positioning the absorbent article.

In one embodiment, the joint is concave. By concave is meant that the joint formed by each side flap together form an hour glass shape. In other words, the joints have a distance between them in the lateral axis of the absorbent article, and that lateral distance narrows and then broadens in the longitudinal direction of the absorbent article.

In one embodiment, the body-facing surface of the chassis comprises a second adhesive to affix the chassis to an upper side of the undergarment during use. In this embodiment, the first adhesive and the second adhesive are arranged to be non-overlapping in use.

In one embodiment, the second surface of each side flap comprises a first adhesive to affix the side flap to the other side flap during use, thereby attaching the absorbent article to the undergarment. In this embodiment, at least one of the side flaps may contain at least one line of perforation to allow release of the absorbent article from the undergarment in use. Preferably, the line of perforation comprises a non-homogeneous perforation pattern to promote easy initiation at removal but prevent accidental separation during use.

In one embodiment, a release liner covers at least a portion of the first adhesive.

In a second aspect, the invention provides a process for preparing an absorbent article as described hereinabove, the process comprising:
providing a chassis and a pair of side flaps, and
bonding the side flaps to the chassis on the body-facing surface to form a joint between each of the side flaps and the body-facing surface of the chassis.

An additional advantage of the absorbent articles of the invention is that they can be manufactured easily in that the wing can be simply laid on top of a standard sanitary napkin without wings and then bonded at the joint. As apparent to the skilled man this allows an easy manufacturing of the article and also allows for manufacturing flexibility as the same line which produces non winged articles can be used to produce winged articles adding a final step of wing bonding to the existing line.

### BRIEF DESCRIPTION OF THE DRAWINGS

While the specification concludes with claims particularly pointing out and distinctly claiming the subject matter of the present invention, it is believed that the invention can be more readily understood from the following description taken in connection with the accompanying drawings, in which:
Figure 1 shows an absorbent article according to the prior art;
Figure 2 shows an absorbent article according to the invention;
Figures 3 and 4 show absorbent articles according to the invention and prior art in use;
Figure 5 shows a side flap according to the invention; and
Figure 6 shows an absorbent article according to the invention.

### DETAILED DESCRIPTION OF THE INVENTION

The present invention may be utilized in absorbent articles as provided below. As used herein, the phrase "absorbent article" refers to devices which absorb and contain body liquids, and more specifically refers to devices which may be placed against or near the skin to absorb and contain the various liquids, such as those discharged from the body. In typical use the absorbent articles are not intended to be laundered or otherwise restored or reused after a single use. Examples of absorbent articles include, but are not limited to: personal care absorbent products, such as: feminine hygiene products, for example feminine sanitary napkins, undergarmentliners, pantyliners, interlabial devices and the like and adult incontinence products.

Absorbent articles, and their individual components, such as a liquid pervious top sheet, a substantially liquid impervious backsheet joined to the topsheet, and an absorbent core disposed between i.e. positioned and held between the topsheet and the backsheet, have a body-facing surface and an outer-facing surface. As used herein, "body-facing surface" means that surface of the article or component which is intended to be disposed toward or placed adjacent to the body of the wearer during ordinary use, while the "outer-facing surface" is on the opposite side, and is intended to be disposed to face away from the wearer's body during ordinary use. The outer-facing surface may be arranged to face toward or placed adjacent to the wearer's undergarments or outer garments when the absorbent article is worn.

The term "nonwoven" or "nonwoven material" refers herein to a material made from continuous (long) filaments (fibers) and/or discontinuous (short) filaments (fibers) by processes such as spunbonding, meltblowing, carding, and the like. Nonwovens do not have a woven or knitted filament pattern.

As used herein, the term "nonwoven web" means a manufactured sheet, web, or batt of directionally or randomly orientated fibers, bonded by friction, and/or cohesion, and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers may have diameters ranging from less than about 0.001 mm to more than about 0.2 mm and may come in several different forms such as short fibers (known as staple, or chopped), continuous single fibers (filaments or monofilaments), untwisted bundles of continuous filaments (tow), and twisted bundles of continuous filaments (yam). Nonwoven webs may be formed by many processes such as meltblowing, spunbonding, solvent spinning, electrospinning, carding, and airlaying. The basis weight of nonwoven webs is usually expressed in grams per square meter (g/m² or gsm).

As used herein, the term "longitudinal" refers herein to a line, axis, or direction in the plane of the disposable absorbent article that is generally aligned with (e.g., approximately parallel to) a vertical plane which bisects a standing wearer into left and right body halves when the article is worn.

As used herein, the terms "transverse" or "lateral" are interchangeable herein and refer to a line, axis, or direction that is perpendicular to the longitudinal direction.

As used herein, the terms "joined", "bonded", or "attached" encompasses configurations whereby an element is directly secured to another element by affixing the element directly to the other element, and configurations whereby an element is indirectly secured to another element by affixing the element to intermediate member(s) which in turn are affixed to the other element.

As used herein, the term hydrophihc , refers to a material having a contact angle less than or equal to 90° according to The American Chemical Society Publication "Contact Angle, Wettability, and Adhesion," edited by Robert F. Gould and copyrighted in 1964.

As used herein, the term "hydrophobic", refers to a material or layer having a contact angle greater than or equal to 90° according to The American Chemical Society Publication "Contact Angle, Wettability, and Adhesion," edited by Robert F. Gould and copyrighted in 1964.

The absorbent articles of the present invention comprise a chassis having a topsheet, a backsheet, and an absorbent core disposed between the topsheet and the backsheet. The absorbent articles further comprise a pair of side flaps attached to the chassis.

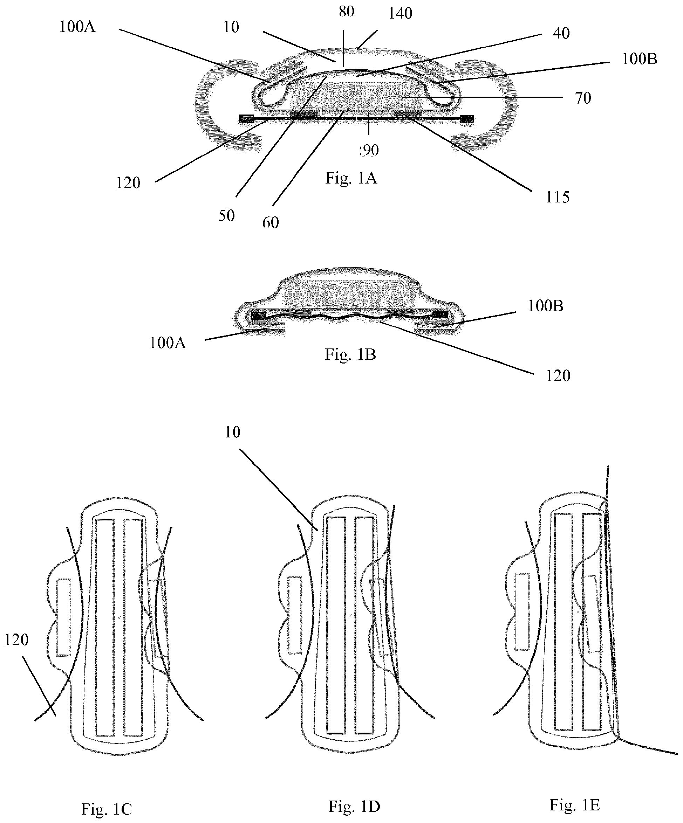

Figure 1A shows a conventional absorbent article 10. When initially removed from its outer packaging, the absorbent article 10 may be folded in half, in thirds or more. The absorbent article 10 comprises a chassis 40 which further comprises a topsheet 50, a backsheet 60, and an absorbent core 70 between the topsheet 50 and the backsheet 60. The absorbent article is shown attached to an undergarment 120 by an adhesive 115 attached to the outer-facing surface 90 of the chassis 40. The absorbent article 10 further comprises a pair of side flaps 100A and 100B which are formed from the chassis 40 (i.e. are unitary with the chassis) and comprise an adhesive which in the folded state is attached to a release liner 140.

Figure 1B shows a conventional absorbent article 10 in which the side flaps 100A and 100B are detached from the release liner and are unfolded (in the direction of the arrows shown in Fig. 1A) and affixed to the underside of undergarment 120.

Figures 1C-1E show the effect of the fold on the undergarment 120. In these figures, the side flaps are folded to a progressively greater extent. In Figure 1C, a small fold is used, which provides a poor fit to the undergarment in the middle of the overlapping region where there is gapping. As the amount of fold is increased in Figures ID and IE, the middle of the overlapping region fits the undergarment more closely and closes the gapping, but then the undergarment is then pinched and constrained in the outer regions of overlap.

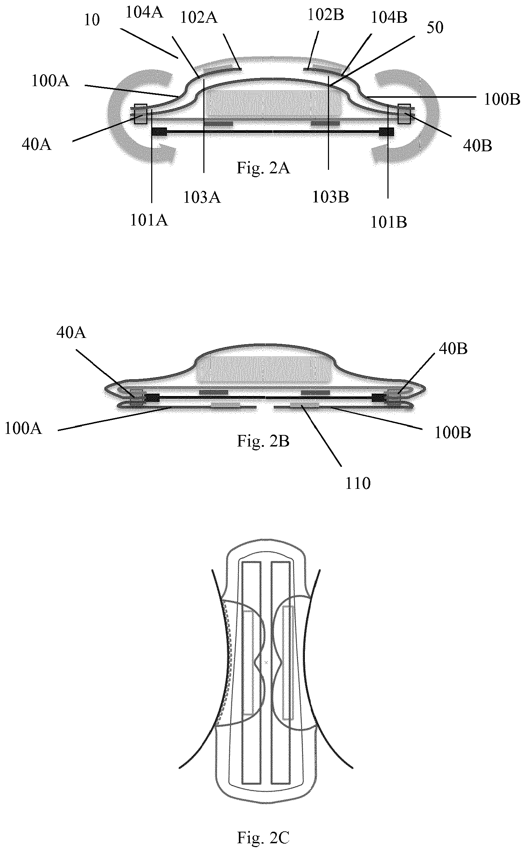

Figures 2A and 2B show an absorbent article 10 according to the invention. In this case, the side flaps 100A and 100B are formed discretely to the chassis 40 and are bonded by their first surface to the chassis on the top sheet 50 at a joint 40.

The side flaps 100A and 100B comprise an attached proximal end 101A and 101B, respectively, and free distal ends 102A and 102B, respectively, and also a first surface 103A and 103B and a second surface 104A and 104B. The proximal ends 101A and 101B are joined to the chassis 40 and form joints 40A and 40B, respectively.

Each of the side flaps 100A and 100B is formed discretely to the chassis and is bonded at the proximal end 101A and 101B by their first surfaces 103A and 103B to the body facing surface 80 of the chassis 40 at a joint 40A and 40B. When presented to the user, each side flap 100A and 100B lies flat on the body facing surface 80 of the absorbent article, and extends inwards towards the centre of the chassis i.e. extends inwards from the joint 40A and 40B in a lateral direction of the absorbent article. Therefore, the first surface 103A and 103B of the side flaps faces the top sheet 50 of the absorbent article.

In use, the free distal ends 102A and 102B are typically positioned subjacent to the undergarment of the user (Figure 2b) such that the adhesive 110 adheres to the undergarment 120.

During the application of the absorbent article 10, the side flaps 100A and 100B can fold about the joint 40A and 40B. The joint facilitates the application of the side flaps 100A and 100B to the undergarment of the wearer. In the side flaps on conventional absorbent articles which have no predefined fold axis, the user is often left guessing or forming, on an ad hoc basis, a fold axis which can introduce stresses into the side flap. Because the wings 100A and 100B are extensible and spaced inwardly of the edge of the chassis, they warp and conform smoothly along the undergarment leg elastic edge without significant gapping (Figure 2c).

The curve may be shaped so as to fit the average undergarment, and in particular female undergarment. Therefore, the crotch dimensions of 90 pairs of female undergarments were measured at the minimum width i.e. in a lateral direction and 60 mm forward (front width) and backward (back width) from the minimum width.

| **Percentile (%)** | **100** | **90** | **50** | **10** | **0** |
|---|---|---|---|---|---|
| Front Width (mm) | 160 | 121 | 93 | 73 | 56 |
| Minimum Width (mm) | 95 | 77 | 66 | 52 | 42 |
| Back Width (mm) | 136 | 121 | 97 | 73 | 28 |

During processing, after creation of the chassis 40, the chassis 40 may be cut to provide the curved first and second longitudinal edges. The side flaps 100A and 100B comprise discrete elements which are attached to the chassis 40 of the absorbent article 10. The side flaps 100A and 100B are not integrally formed from the topsheet 50, the backsheet 60, and/or combinations thereof. The material of each the side flaps 100A and 100B maybe attached to the chassis 40 prior to the cutting process of the chassis 40. So, during the cutting process as mentioned above, the side flaps 100A and 100B, may be subjected to the cutting process as well. The side flaps 100A and 100B may follow the curved periphery of the chassis 40. This can provide the user with additional comfort. Additionally, in such embodiments, many of the stresses placed on conventional side flaps by a user's undergarment are alleviated if not eliminated without additional processing, e.g. stretching, activation, or differential extensibility of nonwoven material for side flaps. Stresses on conventional side flaps are discussed in detail in U.S. Patent No. 5,354,400, issued to Lavash et al.

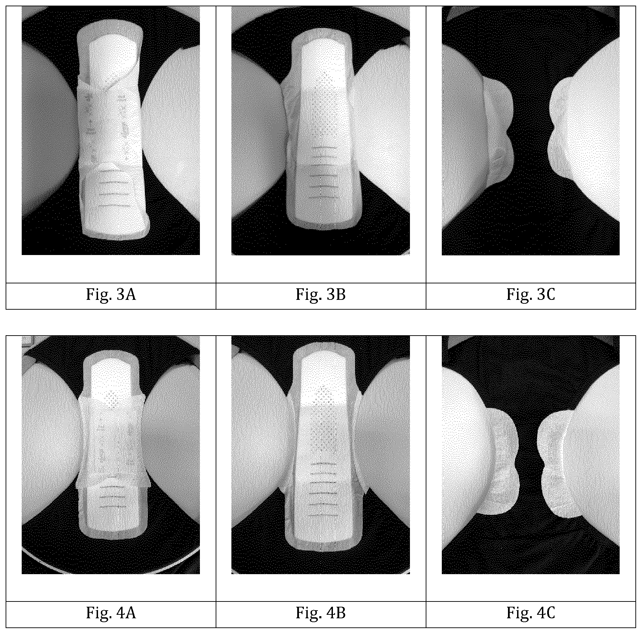

Figures 3 and 4 show, for a conventional absorbent article and an absorbent article according to the invention respectively, initial placement of the absorbent article on the undergarment (Figures 3A/4A) and the article with the side flaps folded around the undergarment from the body side (Figures 3B/4B) and the garment side (Figures 3C/4C). These figures show the improved fit of the absorbent article according to the invention.

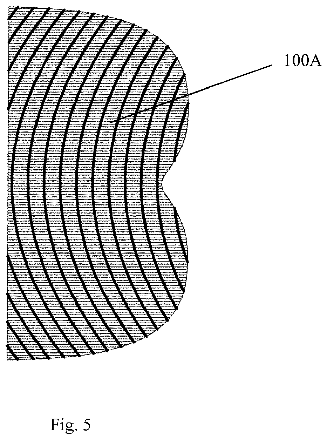

Figure 5 shows a side flap 100A which includes a SELF-pattern so as to be extensible in the longitudinal direction of the absorbent article. The extensibility allows stretch in the region of the side flaps attached to the undergarment, thereby making the undergarment less binding and more comfortable to the wearer. Preferably the side flaps are sufficiently extensible to allow for the elongation in the wing caused by fanning as the wing is folded around the undergarment and also for the maximum elongation of the undergarment at the crotch. Typically, the wings are capable of elongating by 80%.

In this embodiment the SELF-pattern is curved. The pattern contains 1 mm SELF bands that are spaced at 6 mm. The SELF bands are overlaid on an activation pattern with fins spaced at 1mm. An activation depth of 1mm is sufficient to allow a laminate of polyethylene and nonwoven to elongate >80%.

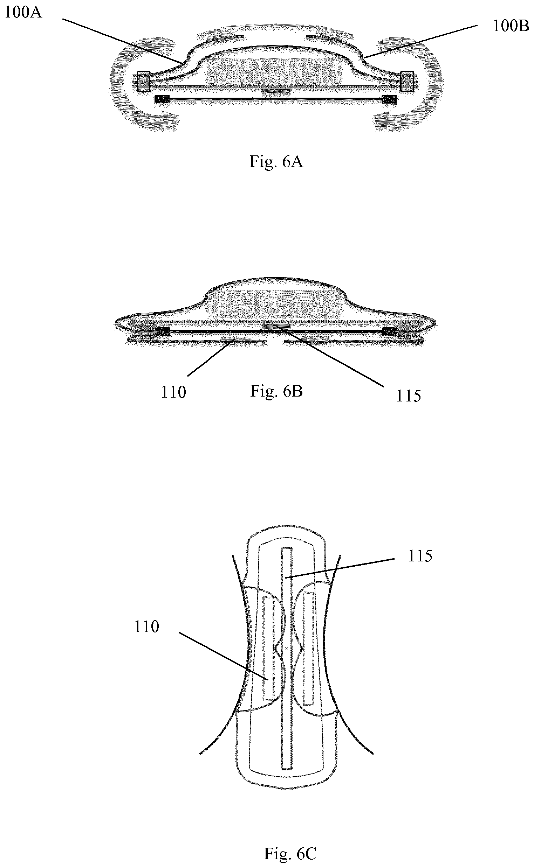

Figure 6 shows an absorbent article 10 wherein the first adhesive 110 on the side flaps 100A and 100B and the second adhesive 115 on the body-facing side of the undergarment are positioned to avoid their overlap when both are affixed to the undergarment. As can be seen by Figure 6C, the first adhesive 110 and second adhesive 115, which are affixed at opposing sides of the undergarment, do not affix the same part of undergarment. This is an advantageous configuration because, since the chassis of the pad is not typically able to stretch in the longitudinal direction, the panty will also not be able to stretch where the chassis adhesive is affixed to it. By concentrating the chassis adhesive in the center of the pad and disposing the wing adhesive so that wings attach more outboard of the chassis adhesive in the transverse direction, without overlapping areas, the wings are not inhibited from stretch in the longitudinal direction when affixed to the panty.

Any suitable material may be utilized for the side flaps disclosed herein. Some examples include but are not limited to, nonwovens, nonwoven laminates, film-nonwoven laminates, elastomeric films laminate, elastomeric nonwovens laminate, elastomeric film-nonwoven laminates, differentially extensible substrates or laminates thereof, textile or woven materials such as cotton fabrics, elastic materials, stretchable materials, and/or combinations thereof. Embodiments are contemplated where at least one of the side flaps comprises an antibacterial and/or antimicrobial additive / composition or bacterial / microbial growth inhibitors. An example of such a composition is described in U.S. Patent No. 7,790,947. For example, silver ions may be added to the material of at least one of the side flaps. In some embodiments, at least one of the side flaps may be breathable allowing air / vapor to permeate therethrough but not allowing liquid to pass therethrough. In some embodiments, at least one of the side flaps may be transparent or translucent or may have portions which are transparent and portions which are translucent. In some embodiments, at least one of the side flaps may comprise a friction reducing composition and / or a lotion and/or a sensate. In some embodiments, the lotion may provide the function of the friction reducing composition in addition to other benefits. Suitable lotions and sensates are disclosed in U.S. Patent Application Publication No. 2012/089110; U.S. Patent Application Publication No. 2011/070277; and U.S. Patent No. 8,357,445. Examples of additional lotions and/or sensates are described in U.S. Pat. No. 6,570,054 and U.S. patent application Ser. No. 12/974,674. In some embodiments, the side flaps may comprise materials which comprise compositions which enhance its hydrophobicity. Some examples include hydrophobic additive formulations. Hydrophobic additive formulations and methods for incorporating them in nonwoven webs are described by Catalan in US applications publication Nos. 2006/0189956 filed on Feb. 18, 2005, and 2005/0177123 filed on Feb. 10, 2005, and in U.S. application Ser. No. 12/691,929 filed on Jan. 22, 2010, and U.S. application Ser. No. 12/691,934 filed on Jan. 22, 2010 both to J J Tee et al. that are all assigned to The Procter and Gamble Company. Some suitable, but not limiting, hydrophobic materials used as hydrophobic surface coatings and/or hydrophobic melt additives may comprise one or more silicone polymers that are also substantially free of aminosilicones. Suitable silicone polymers are selected from the group of silicone MQ resins, polydimethysiloxanes, crosslinked silicones, silicone liquid elastomers, and combinations thereof. Typically, the molecular weight of such silicone polymers should be at least 4000 MW. However, the molecular weight of such silicone polymers may be at least 10,000 MW, at least 15,000 MW, at least 20,000 MW, or at least 25,000 MW. Suitable polydimethylsilosxanes are selected from the group consisting of vinyl-terminated polydimethsiloxanes, methyl hydrogen dimethylsiloxanes, hydroxyl-terminated polydimethysiloxanes, organo-modified polydimethylsiloxanes, and combinations thereof. Alternatively, fluorinated polymers may also be used as the hydrophobic surface coatings and/or the hydrophobic melt additives. Suitable fluorinated polymers are selected from the group of telomers and polymers containing tetrafluoroethylene and/or perfluorinated alkyl chains. For instance, fluorinated surfactants, which are commercially available from Dupont under the tradename Zonyl®, are suitable for use herein.

In some embodiments, the side flaps may comprise "capillary channel fibers." Such fibers can be solid or hollow, and they can be tri-lobal, delta-shaped, and are preferably fibers having capillary channels on their outer surfaces. The capillary channels can be of various cross-sectional shapes such as "U-shaped", "H-shaped", "C-shaped" and "V-shaped". One suitable example of a capillary channel fiber is T-401, designated as 4DG fiber available from Fiber Innovation Technologies, Johnson City, Tenn. T-401 fiber is a polyethylene terephthalate (PET polyester).

In some embodiments, the side flaps may comprise microfibers. Meltblown fibers are microfibers which may be continuous or discontinuous and are generally smaller than 10 microns in average diameter. In some embodiments, the side flaps may comprise Coolmax® fibers.

Any suitable adhesive may be utilized on backsheets and/or side flaps disclosed herein. The adhesive can be hot-melt adhesive material capable of establishing a temporary bond with the undergarment material. A suitable adhesive is the composition designated HL-1491 XZP commercially available from H. B. Fuller, Toronto, Ontario, Canada, a composition designated as H2031 available from Bostik, a composition designated as NS34-2823 as manufactured by National Starch and Chemical of Bridgewater, N.J.

The adhesive can be applied to the backsheet at levels between about 9 gsm to about 20 gsm, in some embodiments. The adhesive applied to the backsheet may be applied in discrete strips or may cover a substantial portion of the backsheet. Regarding the adhesive for the side flaps, the adhesive above may be suitable and an additional adhesive which is suitable is designated as LA203 available from Savare Specialty Adhesives or the composition designated as 1461 available from H.B. Fuller. The adhesive for the side flaps may be applied at levels of between about 13 gsm to about 38 gsm.

Any suitable release liners can be used in conjunction with the present invention. Some examples of suitable release liners include those disclosed in U.S. Patent No. 4,556,146, and U.S. Patent Application Publication No. 2011/0202029. The releasable cover may be a silicone coated release liner, a plastic film or any other easily removable cover. The releasable cover may be in a single piece or in a multitude of pieces, e.g. to cover the individual adhesive areas. It also can perform other functions such as providing individualized packaging for the article or provide a disposal function. Any commercially available release liner or film may be used. Some suitable examples include BL 30 MG-A SILOX EI/O, BL 30 MG-A SILOX 4 P/O available from Akrosil Corporation, and M&W films available from Gronau in Germany, under the code X-5432.

The methods of the invention can be used with any suitable feminine hygiene article or incontinence article. Suitable absorbent articles include any type of structures, from a single absorbent layer to more complex multi layer structures. Certain absorbent articles typically include a fluid pervious topsheet, a backsheet, which may be fluid impervious and/or may be water vapour and/or gas pervious, and an absorbent element often called "core" comprised there between.

In general the topsheet is operatively permeable to the liquids that are intended to be held or stored by the absorbent article, and the backsheet maybe substantially impermeable or otherwise operatively impermeable to the intended liquids. The absorbent article may also include other components, such as a secondary topsheet, liquid wicking layers, liquid distribution layers, barrier layers, and the like, as well as combinations thereof. Suitable topsheets, backsheets, side flaps and absorbent core materials for use in conjunction with the present invention are discussed hereafter. Additionally, suitable joining methods for topsheets, backsheets, side flaps, and optionally the absorbent core are also discussed hereafter.

The topsheet may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be included of natural fibers (e.g., wood or cotton fibers), synthetic fibers (e.g., polymeric fibers such as polyester, polypropylene, or polyethylene fibers) or from a combination of natural and synthetic fibers. In one embodiment, the topsheet may be made of a hydrophobic material to isolate the wearer's skin from liquids which have passed through the topsheet. If the topsheet is made of a hydrophobic material, at least the upper surface of the topsheet is treated to be hydrophilic so that liquids will transfer through the topsheet more rapidly. This diminishes the likelihood that body exudates will flow off the topsheet rather than being drawn through the topsheet and being absorbed by the absorbent core. In one embodiment, the topsheet can be rendered hydrophilic by treating it with a surfactant. Suitable methods for treating the topsheet with a surfactant include spraying the topsheet material with the surfactant and immersing the material into the surfactant.

The topsheet can include an apertured formed film. Apertured formed films can be used for the topsheet because they are pervious to body exudates and yet non-absorbent and have a reduced tendency to allow liquids to pass back through and rewet the wearer's skin. Thus, the surface of the formed film which is in contact with the body remains dry, thereby reducing body soiling and creating a more comfortable feel for the wearer. Suitable formed films are described in U.S. Pat. No. 3,929,135, entitled "Absorptive Structures Having Tapered Capillaries", issued to Thompson on Dec. 30, 1975; U.S. Pat. No. 4,324,246 entitled "Disposable Absorbent Article Having A Stain Resistant Topsheet", issued to Mullane, et al. on Apr. 13, 1982; U.S. Pat. No. 4,342,314 entitled "Resilient Plastic Web Exhibiting Fiber-Like Properties", issued to Radel, et al. on Aug. 3, 1982; U.S. Pat. No. 4,463,045 entitled "Macroscopically Expanded Three-Dimensional Plastic Web Exhibiting Non-Glossy Visible Surface and Cloth-Like Tactile Impression", issued to Ahr, et al. on Jul. 31, 1984; and U.S. Pat. No. 5,006,394 "Multilayer Polymeric Film" issued to Baird on Apr. 9, 1991.

The absorbent core can be any absorbent member which is generally compressible, conformable, non-irritating to the wearer's skin, and capable of absorbing and retaining body fluids. The absorbent core may be manufactured in a wide variety of sizes and shapes (e.g., rectangular, hourglass, "T" -shaped, asymmetric, etc.) and from a wide variety of liquid-absorbent materials commonly used in disposable pull-on garments and other absorbent articles such as commuted wood pulp which is generally referred to as airfelt. Examples of other suitable absorbent materials include creped cellulose wadding; meltblown polymers including coform; chemically stiffened, modified or cross-linked cellulosic fibers; tissue including tissue wraps and tissue laminates; absorbent foams; absorbent sponges; superabsorbent polymers; absorbent gelling materials; or any equivalent material or combinations of materials.

The configuration and construction of the absorbent core may vary (e.g., the absorbent core may have varying caliper zones, a hydrophilic gradient, a superabsorbent gradient, or lower average density and lower average basis weight acquisition zones; or may include one or more layers or structures). Further, the size and absorbent capacity of the absorbent core may also be varied to accommodate wearers ranging from infants through adults. However, the total absorbent capacity of the absorbent core should be compatible with the design loading and the intended use of the absorbent article.

The absorbent core may include other optional components. One such optional component is the core wrap, i.e., a material, typically but not always a nonwoven material, which either partially or totally surrounds the core. Suitable core wrap materials include, but are not limited to, cellulose, hydrophilically modified nonwoven materials, perforated films and combinations thereof.

The backsheet can comprise a liquid impervious film. The backsheet can be impervious to liquids (e.g., body fluids) and can be typically manufactured from a thin plastic film. However, typically the backsheet can permit vapours to escape from the disposable article. In an embodiment, a microporous polyethylene film can be used for the backsheet. A suitable microporous polyethylene film is manufactured by Mitsui Toatsu Chemicals, Inc., Nagoya, Japan and marketed in the trade as PG-P.

One suitable material for the backsheet can be a liquid impervious thermoplastic film having a thickness of from about 0.012 mm (0.50 mil) to about 0.051 mm (2.0 mils), for example including polyethylene or polypropylene. Typically, the backsheet can have a basis weight of from about 5 g/m² to about 35 g/m². However, it should be noted that other flexible liquid impervious materials may be used as the backsheet. Herein, "flexible" refers to materials which are compliant and which will readily conform to the general shape and contours of the wearer's body.

The backsheet can be typically positioned adjacent a outer-facing surface of the absorbent core and can be joined thereto by any suitable attachment device known in the art. For example, the backsheet may be secured to the absorbent core by a uniform continuous layer of adhesive, a patterned layer of adhesive, or an array of separate lines, spirals, or spots of adhesive. Illustrative, but non-limiting adhesives, include adhesives manufactured by H. B. Fuller Company of St. Paul, Minn., U.S.A., and marketed as HL-1358J. An example of a suitable attachment device including an open pattern network of filaments of adhesive is disclosed in U.S. Pat. No. 4,573,986 entitled "Disposable Waste-Containment Garment", which issued to Minetola et al. on Mar. 4, 1986. Another suitable attachment device including several lines of adhesive filaments swirled into a spiral pattern is illustrated by the apparatus and methods shown in U.S. Pat. No. 3,911,173 issued to Sprague, Jr. on Oct. 7, 1975; U.S. Pat. No. 4,785,996 issued to Ziecker, et al. on Nov. 22, 1978; and U.S. Pat. No. 4,842,666 issued to Werenicz on Jun. 27, 1989. Alternatively, the attachment device may include heat bonds, thermal fusion bonds, pressure bonds, ultrasonic bonds, dynamic mechanical bonds, or any other suitable attachment device or combinations of these attachment devices. The backsheet may be additionally secured to the topsheet by any of the above-cited attachment devices / methods.

The absorbent article may also include such other suitable features as are known in the art including, but not limited to, re-closable fastening system, lotion, acquisition layers, distribution layers, wetness indicators, sensors, elasticized waist bands and other similar additional elastic elements and the like, belts and the like, waist cap features, containment and aesthetic characteristics and combinations thereof.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. An absorbent article (10) comprising:
a chassis (40) comprising a topsheet (50), a backsheet (60), and an absorbent core (70) disposed between the topsheet and the backsheet, the chassis having a body-facing surface (80) and an outer-facing surface (90), and
a pair of side flaps (100A, 100B) comprising a first surface (103A, 103B), a second surface (104A, 104B) opposite the first surface, and a first adhesive (110),
each of the side flaps having a proximal end (101A, 101B) and a free distal end (102A, 102B),
wherein each distal end is foldable around an edge of an undergarment (120) when applied on the undergarment during use to attach the first adhesive to the underside of the undergarment thereby attaching the absorbent article to the undergarment,
wherein each of the side flaps is formed discretely to the chassis and is bonded at the proximal end by the first surface to the body facing surface of the chassis at a joint (40A and 40B),
and wherein each side flap extends inwards from the joint in a lateral direction of the absorbent article with the first surface facing the top sheet of the absorbent article,
and wherein each side flap is extensible.

2. The absorbent article according to claim 1, wherein the chassis has a lateral edge and the joint is spaced from the lateral edge over at least a part of a longitudinal extent of the joint.

3. The absorbent article according to claim 2, wherein the joint is spaced from the lateral edge over all the longitudinal extent of the joint.

4. The absorbent article according to claim 3, wherein the joint is substantially parallel to the lateral edge.

5. The absorbent article according to any one of claims 2 to 4, wherein the joint is spaced from the lateral edge by between 3 mm and 10 mm.

6. The absorbent article according to any one of claims 1 to 5, wherein the side flaps are bonded to the body facing surface mechanically, ultrasonically, thermally, by pressure, cohesively or adhesively.

7. The absorbent article according to any one of claims 1 to 6, wherein the side flaps are bonded to the body-facing surface in a manner which is continuous or intermittent.

8. The absorbent article according to any one of claims 1 to 7, wherein the side flaps are extensible in a longitudinal direction of the absorbent article.

9. The absorbent article according to any one of claims 1 to 8, wherein the side flaps are made extensible by mechanical activation.

10. The absorbent article according to any one of claims 1 to 9, wherein the joint is curved.

11. The absorbent article according to claim 10, wherein the joint is concave.

12. A process for preparing an absorbent article according to any one of claims 1 to 11, the process comprising:
providing a chassis and a pair of side flaps, and
bonding the side flaps to the chassis on the body-facing surface to form a joint between each of the side flaps and the body-facing surface of the chassis.

## Patentansprüche

1. Absorptionsartikel (10), umfassend:
eine Grundeinheit (40), umfassend eine obere Lage (50), eine untere Lage (60) und einen Absorptionskern (70), der zwischen der oberen Lage und der unteren Lage angeordnet ist, wobei die Grundeinheit eine dem Körper zugewandte Fläche (80) und eine der Außenseite zugewandte Fläche (90) aufweist, und
ein Paar Seitenklappen (100A, 100B), welche eine erste Fläche (103A, 103B), eine zweite Fläche (104A, 104B) gegenüber der ersten Fläche und einen ersten Klebstoff (110) umfassen,
wobei jede der Seitenklappen ein proximales Ende (101A, 101B) und ein freies distales Ende (102A, 102B) aufweist,
wobei jedes distale Ende um einen Rand einer Unterwäsche (120) faltbar ist, wenn es während der Verwendung auf die Unterwäsche aufgebracht wird, um den ersten Klebstoff an der Unterseite der Unterwäsche zu befestigen, wodurch der Absorptionsartikel an der Unterwäsche befestigt wird,
wobei jede der Seitenklappen separat zu der Grundeinheit ausgebildet ist und am proximalen Ende durch die erste Fläche an einer Verbindungsstelle (40A und 40B) mit der dem Körper zugewandten Fläche der Grundeinheit verbunden ist,
und wobei sich jede Seitenklappe von der Verbindungsstelle in einer Querrichtung des Absorptionsartikels nach innen erstreckt, wobei die erste Fläche der oberen Lage des Absorptionsartikels zugewandt ist,
und wobei jede Seitenklappe dehnbar ist.

2. Absorptionsartikel nach Anspruch 1, wobei die Grundeinheit einen Seitenrand aufweist und die Verbindungsstelle über zumindest einen Teil einer Längsausdehnung der Verbindungsstelle einen Abstand von dem Seitenrand aufweist.

3. Absorptionsartikel nach Anspruch 2, wobei die Verbindungsstelle über die gesamte Längsausdehnung der Verbindungsstelle einen Abstand von dem Seitenrand aufweist.

4. Absorptionsartikel nach Anspruch 3, wobei die Verbindungsstelle im Wesentlichen parallel zu dem Seitenrand angeordnet ist.

5. Absorptionsartikel nach einem der Ansprüche 2 bis 4, wobei die Verbindungsstelle einen Abstand von 3 mm bis 10 mm von dem Seitenrand aufweist.

6. Absorptionsartikel nach einem der Ansprüche 1 bis 5, wobei die Seitenklappen mechanisch, durch Ultraschall, thermisch, durch Druck, kohäsiv oder adhäsiv mit der dem Körper zugewandten Fläche verbunden sind.

7. Absorptionsartikel nach einem der Ansprüche 1 bis 6, wobei die Seitenklappen auf eine Weise mit der dem Körper zugewandten Fläche verbunden sind, die kontinuierlich oder unterbrochen ist.

8. Absorptionsartikel nach einem der Ansprüche 1 bis 7, wobei die Seitenklappen in einer Längsrichtung des Absorptionsartikels dehnbar sind.

9. Absorptionsartikel nach einem der Ansprüche 1 bis 8, wobei die Seitenklappen durch mechanische Aktivierung dehnbar gemacht werden.

10. Absorptionsartikel nach einem der Ansprüche 1 bis 9, wobei die Verbindungsstelle gekrümmt ist.

11. Absorptionsartikel nach Anspruch 10, wobei die Verbindungsstelle konkav ist.

12. Verfahren zur Herstellung eines Absorptionsartikels nach einem der Ansprüche 1 bis 11, wobei das Verfahren umfasst:
Bereitstellen einer Grundeinheit und eines Paars Seitenklappen, und
Verbinden der Seitenklappen mit der Grundeinheit auf der dem Körper zugewandten Fläche, um eine Verbindungsstelle zwischen jeder der Seitenklappen und der dem Körper zugewandten Fläche der Grundeinheit zu bilden.

## Revendications

1. Article absorbant (10) comprenant :
un châssis (40) comprenant une feuille de dessus (50), une feuille de fond (60), et une âme absorbante (70) disposée entre la feuille de dessus et la feuille de fond, le châssis ayant une surface faisant face au corps (80) et une surface tournée vers l'extérieur (90), et
une paire de rabats latéraux (100A, 100B) comprenant une première surface (103A, 103B), une deuxième surface (104A, 104B) opposée à la première surface, et un premier adhésif (110),
chacun des rabats latéraux ayant une extrémité proximale (101A, 101B) et une extrémité distale libre (102A, 102B),
dans lequel chaque extrémité distale est pliable autour d'un bord d'un sous-vêtement (120) lorsqu'il est appliqué sur le sous-vêtement pendant l'utilisation pour fixer le premier adhésif à la surface inférieure du sous-vêtement fixant de ce fait l'article absorbant au sous-vêtement,
dans lequel chacun des rabats latéraux est formé de manière distincte sur le châssis et est lié au niveau de l'extrémité proximale par la première surface à la surface faisant face au corps du châssis au niveau d'une articulation (40A et 40B),
et dans lequel chaque rabat latéral s'étend vers l'intérieur à partir de l'articulation dans une direction latérale de l'article absorbant avec la première surface faisant face à la feuille de dessus de l'article absorbant,
et dans lequel chaque rabat latéral est extensible.

2. Article absorbant selon la revendication 1, dans lequel le châssis a un bord latéral et l'articulation est espacée du bord latéral sur au moins une partie d'une étendue longitudinale de l'articulation.

3. Article absorbant selon la revendication 2, dans lequel l'articulation est espacée du bord latéral sur toute l'étendue longitudinale de l'articulation.

4. Article absorbant selon la revendication 3, dans lequel l'articulation est sensiblement parallèle au bord latéral.

5. Article absorbant selon l'une quelconque des revendications 2 à 4, dans lequel l'articulation est espacée du bord latéral de 3 mm à 10 mm.

6. Article absorbant selon l'une quelconque des revendications 1 à 5, dans lequel les rabats latéraux sont liés à la surface faisant face au corps de façon mécanique, par ultrasons, de façon thermique, par pression, de façon cohérente ou de façon adhésive.

7. Article absorbant selon l'une quelconque des revendications 1 à 6, dans lequel les rabats latéraux sont liés à la surface faisant face au corps d'une manière qui est continue ou intermittente.

8. Article absorbant selon l'une quelconque des revendications 1 à 7, dans lequel les rabats latéraux sont extensibles dans une direction longitudinale de l'article absorbant.

9. Article absorbant selon l'une quelconque des revendications 1 à 8, dans lequel les rabats latéraux sont rendus extensibles par activation mécanique.

10. Article absorbant selon l'une quelconque des revendications 1 à 9, dans lequel l'articulation est courbée.

11. Article absorbant selon la revendication 10, dans lequel l'articulation est concave.

12. Procédé de préparation d'un article absorbant selon l'une quelconque des revendications 1 à 11, le procédé comprenant :
la fourniture d'un châssis et d'une paire de rabats latéraux, et
la liaison des rabats latéraux au châssis sur la surface faisant face au corps pour former une articulation entre chacun des rabats latéraux et la surface faisant face au corps du châssis.
